# NEUE EUROPÄISCHE PATENTSCHRIFT

(11) **EP 0 485 840 B2**
(45) Veröffentlichungstag und Bekanntmachung des Hinweises auf die Entscheidung über den Einspruch: **03.03.1999**
(45) Hinweis auf die Patenterteilung: 08.03.1995
(21) Anmeldenummer: 91118734.2
(22) Anmeldetag: 04.11.1991
(51) Int. Cl.: A61K 9/36, A61K 9/52

(54) **Orale Arzneiform und Überzugsmittel, enthaltend ein im Colon abbaubares Polysaccharid**
Pharmaceutical oral composition and coating agent containing a polysaccharide degradable in the colon
Composition pharmaceutique orale et agent d'enrobage, contenant un polysaccharide dégradable dans le colon

(30) Priorität: 14.11.1990 DE 9015551 U
(43) Veröffentlichungstag der Anmeldung: 20.05.1992
(73) Patentinhaber: Röhm GmbH, 64293 Darmstadt (DE)
(72) Erfinder: Lehmann, Klaus, Dr., W-6101 Rossdorf 1 (DE); Dreher, Dieter, W-6103 Griesheim (DE); De Pascali, Giuseppe, W-6108 Weiterstadt (DE)

(56) Entgegenhaltungen:
- EP-A- 148 811
- EP-A- 0 152 038
- EP-A- 0 355 536
- EP-A- 0 462 003
- WO-A-83/00435
- DD-A- 221 365
- DD-A- 291 667
- DD-A- 291 668
- DE-A- 3 046 559
- US-A- 4 910 021
- Pharm. Ind. 34 Nr 11a (1972) pp. 892-894
- Ullmanns Encyklopädie der technischen Chemie, Band 18, 1967, pp. 548, 552, 565
- Römpp Chemie Lexikon, 1989, p. 340
- Bauer et al "Uberzogene Arzneiformen", pp. 236-244, Wissenschaftliche VerlagsgesellschaftmbH, Stuttgart, 1988

## Beschreibung

Die Erfindung betrifft eine zur Wirkstofffreigabe im Colon bestimmte orale Arzneiform, enthaltend wenigstens einen Wirkstoff und wenigstens ein, den Wirkstoff umschließendes Hüllmaterial, das ein im Colon abbaubares Polysaccharid, das wesentliche Anteile an Galactose- und Mannose-Einheiten aufweist, enthält, ausgenommen Mikrokapseln, die in dem Hüllmaterial eine ölige Flüssigkeit eingeschlossen enthalten, dadurch gekennzeichnet, daß das Hüllmaterial in Mischung mit dem Polysaccharid ein filmbildendes Polymermaterial enthält.

### Stand der Technik

Es ist allgemein bekannt, orale Arzneiformen mit magensaftbeständigen Überzügen zu versehen, um den enthaltenen Wirkstoff erst beim Eintritt der Arzneiform in den Darm freizusetzen. Für diesen Zweck sind zahlreiche Überzugsmittel bekannt, wie Celluloseacetatphthalat (CAP), Celluloseacetat-trimellitsäure (CAT), Hydroxypropyl-Celluloseacetatphthalat (HPMCP), Carboxymethylethylcellulose (DMEC), Polyvinylacetatphthalat (PVAP) und Copolymere von Methacrylsäure mit Acryl- und/oder Methacrylsäureestern (Handelsbezeichnung EUDRAGIT^{(R)}-L und -S, Röhm Pharma GmbH).

In der EP-B 152 038 ist angegeben, wie man durch Abmischung eines bei pH-Werten von 5 bis 8 wasserlöslichen, carboxylgruppenhaltigen Polymeren mit einem nicht wasserlöslichen, filmbildenden Polymeren durch einen zunehmenden Anteil des letzteren die Wirkstofffreisetzung immer weiter in die unteren Darmabschnitte verlagern kann, Dabei spielen sowohl die Dauer des Aufenthalts der Arzneiform im Darmsaft als auch dessen zunehmender pH-Wert eine Rolle, Es wird eine Überzugsmischung angegeben, die den Wirkstoff bei einem pH-Wert von 7,5 erst nach mehr als drei Stunden freisetzt.

Bei der Steuerung der Wirkstofffreisetzung allein nach dem im Colon erwarteten pH-Wert kommt es häufig in Abhängigkeit von der Konstitution des Patienten und seinen Eß- und Lebensgewohnheiten zu erheblichen Schwankungen des Freisetzungsprofils. Der Wirkstoff kann früher oder später als erforderlich freigegeben werden oder sogar ungenutzt mit den Faeces abgehen. Die gezielte Wirkstofffreisetzung im Colon bereitet aus diesen Gründen Schwierigkeiten. Man ist daher bemüht, neben der Aufenthaltsdauer im Darmtrakt und dem pH-Wert sich weitere Umgebungsfaktoren zunutze zu machen, um den Wirkstoff gezielt im Colon freizusetzen.

Nach C.M. Lancaster und M.A. Wheatley, Polym.Prepr. (American Chemical Society, Division Polymer Chemistry) 1989, 30(1), Seiten 480-481, unterscheidet sich das Colon von den höheren Darmabschnitten durch eine stärkere Bakterienflora. Viele der dort vorkommenden Bakterien erzeugen glykosidische Enzyme, wie beta-Glukuronidase, beta-Xylosidase, alpha- oder beta-Glukosidase, beta-Galactosidase, Mannanase und Galactomannanase durch die bestimmte natürliche Polysaccharide abgebaut werden, Die Autoren haben daher versucht, orale Arzneiformen mit einem im Colon abbaubaren Polysaccharid zu überziehen, das in den oberen Darmabschnitten ungelöst bleibt, so daß es erst im Colon zu einer Auflösung des Überzugs und zur Freisetzung des Wirkstoffes kommt. Es zeigte sich jedoch, daß die überzogenen Arzneiformen bereits in den oberen Darmabschnitten rasch zerfielen. Dies wurde auf die unzureichende Filmbildungsfähigkeit der verwendeten Polysaccharide und die daraus folgende ungenügende mechanische Beständigkeit zurückgeführt. Versuche, die Überzüge durch Weichmachungsmittel elastischer und beständiger zu machen, blieben erfolglos. Triacetin, Citronensäureester, Polyethylenglykole und Glycerin hatten nicht die erhoffte Wirkung. Daher wurde ein weiterhin bestehender Bedarf an geeigneten Weichmachungsmitteln oder nach Methoden zur ionischen oder chemischen Vernetzung der Polysaccharide festgestellt.

K.H. Bauer (Deutsche Apotheker-Zeitung, 130.Jg, Seite 1172 vom 17.5. 1990) hat Polyurethan-Oligosaccharid-Blockpolymere als Überzugsmittel für orale Arzneiformen entwickelt, die magensaftresistent sind und im Dünndarm nicht abgebaut werden. Erst die glykolytische Aktivität der Dickdarmflora spaltet das Überzugsmittel auf, Es ist dadurch möglich, Arzneistoffe, wie z.B. Peptide, die im Magen oder Dünndarm zerstört würden, unzersetzt bis in den Dickdarm zu transportieren. Die toxikologische Prüfung dieser Überzugsmittel steht noch aus.

Aus der EP-A 355 536 sind wirkstoffhaltige Gelfilme bekannt, die ein filmbildendes Polymermaterial und ein Polysaccharid enthalten können. Es handelt sich jedoch nicht um orale Arzneimittel.

Gemäß der nicht vorveröffentlichten EP-A 462 003 werden Mikrokapseln mit einer öligen Flüssigkeit und einer Mischung von filmbildenden Polymermaterialien und Polysacchariden als Hüllmaterial erzeugt.

### Aufgabe und Lösung

Der Erfindung liegt die Aufgabe zugrunde, eine zur Wirkstoffabgabe im Colon bestimmte, orale Arzneiform bzw. ein filmbildendes Überzugsmittel für eine solche orale Arzneiformen auf der Basis von im Colon abbaubaren Polysacchariden zu schaffen, die erst unter der Einwirkung glykosidischer Enzyme abgebaut werden, eine ausreichende mechanische Festigkeit ergeben und aus toxikologisch und pharmakologisch unbedenklichen Bestandteilen aufgebaut sind.

Es wurde gefunden, daß die Aufgabe durch eine zur Wirkstofffreigabe im Colon bestimmte orale Arzneiform, enthaltend wenigstens einen Wirkstoff und wenigstens ein, den Wirkstoff umschließendes Hüllmaterial, das ein im Colon abbaubares Polysaccharid, das wesentliche Anteile an Galactose- und Mannose-Einheiten aufweist, enthält, ausgenommen Mikrokapseln, die in dem Hüllmaterial eine ölige Flüssigkeit eingeschlossen enthalten, dadurch gekennzeichnet, daß das Hüllmaterial in Mischung mit dem Polysaccharid ein filmbildendes Polymermaterial enthält. gelöst wird. Gegenstand der Erfindung sind neben der oralen Arzneiform auch das Überzugsmittel für Arzneimittel, enthaltend eine flüssige Phase, ein im Colon abbaubares Polysaccharid und ein filmbildendes Polymermaterial, das in Form von Latexteilchen in der flüssigen Phase dispergiert ist.

Überraschenderweise ergibt die Mischung auch dann gleichmäßige, mechanisch beständige Überzugsfilme, wenn das Polysaccharid, das selbst nicht ausreichend filmbildend ist, den überwiegenden Anteil des Films ausmacht.

Als Hüllmaterial werden Bestandteile der Arzneiformen bezeichnet, welche den Wirkstoff einschließen und bis zu ihrer Auflösung die Freisetzung des Wirkstoffes hemmen oder unterbinden. Das Hüllmaterial kann zum Beispiel einen den Wirkstoff enthaltenden Kern in Form eines Überzugs umschließen. Es kann jedoch auch eine Matrix bilden, in der der Wirkstoff homogen oder in Form von Kristallen oder eines Granulats verteilt ist.

Im Colon abbaubare Polysaccharide, die pharmakologisch unbedenklich sind, stehen für die Ausführung der Erfindung in ausreichender Zahl und Qualität zur Verfügung. Ebenso sind zahlreiche filmbildende, magensaftbeständige Polymermaterialien, die als Überzugsmittel für orale Arzneiformen geeignet und pharmakologisch unbedenklich sind, bekannt und im Handel erhältlich. Die Kombination dieser Bestandteile ist in toxikologischer und pharmakologischer Hinsicht ebenso unbedenklich wie ihre Bestandteile selbst. Sie führt erfindungsgemäß zu Arzneiformen, die die Anforderungen an die mechanische Beständigkeit erfüllen und die gezielte Freisetzung von Wirkstoffen im Colon ermöglichen.

Daher wird durch die Erfindung in der Palette der Überzugsmittel für Arzneiformen eine Lücke geschlossen, die im Bereich der colon-löslichen Überzüge bestanden und die galenischen Möglichkeiten eingeengt hatte.

### Das Hüllmaterial

Im Colon abbaubare Polysaccharide sind in der eingangs zitierten Veröffentlichung von Lancaster und Wheatley beschrieben. Vorzugsweise handelt es sich um Polysaccharide, die durch glykosidisches Enzyme, insbesondere aus der Gruppe aus beta-Glukuronidase, beta-Xylosidase, alpha- oder beta-Glukosidase und beta-Galactosidase, abbaubar sind. Johannisbrotkernmehl und Guar Gum werden am besten abgebaut, weshalb diese Polysaccharide bevorzugt sind. Erfindungsgemäß sind Polysaccharide, die wesentliche Anteile, vorzugsweise etwa 20 bis 100 Gew.-%, an Galactose- und Mannose-Einheiten enthalten, geeignet.

Als filmbildende Polymermaterialien eignen sich Stoffe, die sich bereits als Überzugsmittel für orale Arzneiformen bewährt haben. Sie sind als filmbildend zu bezeichnen, wenn ihre Lösungen oder Dispersionen zu zusammenhängenden, trocknen, nicht klebrigen, glänzenden Filmen auftrocknen. Wichtig ist, daß sie eine ausreichende Härte mit der erforderlichen Zähigkeit, Dehnbarkeit und Abriebfestigkeit verbinden. Mitunter werden diese Eigenschaften erst bei Zusatz von Weichmachungsmitteln, wie Triacetin, Citronensäureestern, Polyethylenglykolen und Glycerin, erreicht.

Im Rahmen der Erfindung erfüllt das filmbildende Polymermaterial die Aufgabe, das allein nicht ausreichend filmbildende Polysaccharid so in einer galenisch brauchbaren Schicht anzuordnen, daß es durch glykosidische Enzyme angreifbar ist und die gezielte Freisetzung des Wirkstoffes im Colon gewährleistet. Diese Schicht hat die Eigenschaft, im Darmsaft - auch in den oberen Dünndarmabschnitten - zu quellen, ohne daß es zu einer Auflösung der Schicht kommt. Die Diffusion des Wirkstoffes durch die gequollene Schicht geht vor Erreichen des Colon über 10 % der Dosismenge nicht hinaus, was pharmakologisch meistens akzeptabel ist. Erforderlichenfalls kann eine unerwünschte vorzeitige Diffusion durch eine Isolierschicht aus einem magensaftresistenten galenischen Überzugsmittel zwischen dem wirkstoffhaltigen Kern und der quellbaren Schicht unterdrückt werden. Die gequollene Schicht wird erst abgebaut und der eingeschlossene Wirkstoff zugänglich gemacht, wenn im Colon glykosidische Enzyme einwirken.

Die Freisetzung von Wirkstoff schon im Magensatt ist grundsätzlich nicht erwünscht. Es ist deshalb von Vorteil, ein filmbildendes Polymermaterial zu verwenden, das im Magensaft unlöslich und nicht wesentlich quellbar, aber im Darmsalt bei pH-Werten über 5 löslich oder wenigstens quellbar ist. Die Quellung beginnt erst dann, wenn der Darmsaft auf das Polymermaterial einwirkt. Ein im Magensaft lösliches filmbildendes Polymermaterial kann verwendet werden, wenn die erfindungsgemäße Schicht mit einer zusätzlichen Schicht aus einem magensaftresistenten, darmsaftlöslichen galenischen Überzugsmittel überdeckt wird. Die Quellung setzt dann erst ein, wenn der Magen durchlaufen und der zusätzliche Überzug durch den Darmsaft abgelöst worden ist.

Die Quellung der Polysaccharid enthaltenden Schicht wird gefördert, wenn das filmbildende Polymermaterial hydrophile Gruppen enthält, z.B. Carboxyl-, Carboxylat-, tertiäre Amino-, quartäre Ammonium- oder Hydroxylgruppen.

Eine bevorzugte Gruppe von filmbildenden Polymeren sind Acrylpolymere. Sie sind vorzugsweise zu 30 bis 100 Gew.-% aus Monomereinheiten von niederen Alkylestern der Acryl- und/oder Methacrylsäure, insbesondere mit 1 bis 4 Kohlenstoffatomen im Alkylrest, und gegebenenfalls bis zu 70 Gew.-% aus funktionalen Monomereinheiten von äthylenisch ungesättigten, radikalisch polymerisierbaren Monomeren mit einer hydrophilen Gruppe aufgebaut. Acrylpolymere, die ausschließich aus Alkylestern der Acryl- und/oder Methacrylsäure aufgebaut sind oder höchstens geringe, 5 Gew.-% nicht überschreitende Anteile an Acryl- und/oder Methacrylsäure enthalten, sind weder im Magennoch im Darmsatt löslich. Sie ergeben jedoch in der erfindungsgemäßen Kombination Umhüllungen, die im Colon diffusionsdurchlässig werden oder zerfallen.

Mit zunehmenden Gehalt an funktionalen Monomeren nimmt die Löslichkeit der Polymeren im Darmsatt zu. Soweit die funktionalen Gruppen Carboxyl- oder Carboxylat-Gruppen sind, steigt die Lösungsgeschwindigkeit mit zunehmendem pH-Wert an. In der erfindungsgemäßen Kombination mit Polysacchariden ist die Beständigkeit der Schicht gegen Verdauungssäfte trotz starker Quellung insgesamt größer als die des Acrylpolymeren allein. Daher eignen sich für das Hüllmaterial der Erfindung auch solche filmbildenden Polymeren, die sich bei alleiniger Anwendung schon in den oberen Darmabschnitten lösen würden. Auch filmbildende Polymere mit tertiären Aminogruppen, die im pH-Bereich des Darmes oberhalb 5 unlöslich, aber permeabel sind, können verwendet werden, wenn sie während der Magenpassage durch einen magensaftresistenten Überzug vor der Auflösung geschützt sind.

Der Anteil der funktionalen Monomeren mit Carboxyl- bzw. Carboxylatgruppen in den Acrylpolymeren beträgt vorzugsweise 30 bis 50 Gew.-%; derjenige der Monomeren mit tertiären Amino- oder quartären Ammoniumgruppen vorzugsweise 5 bis 10 Gew.-%, bezogen auf das Gesamtgewicht des Polymerisats.

Die funktionalen Monomereinheiten des Acrylpolymeren leiten sich vorzugsweise von Acryl- und/oder Methacrylsäure oder deren Derivaten ab. Dazu gehören die Salze der Acryl- und/oder Methacrylsäure, insbesondere die Alkali- oder Ammoniumsalze, sowie deren Ester oder N-substituierten Amide, die eine Hydroxylgruppe oder eine tertiäre Amino- oder quartäre Ammonium-Gruppe im Esterrest oder im N-Substituenten enthalten. Bevorzugte Hydroxyalkylester sind 2-Hydroxyethyl-acrylat und -methacrylat, 2-Hydroxypropyl-acrylat und -methacrylat und Diethylenglykol-monoacrylat und -monomethacrylat. Beispiele Aminogruppen enthaltender Monomeren sind:
2-Dimethylamino-ethyl-acrylat und -methacrylat
3-(N,N-Dimethylamino)-propyl-acrylat und -methacrylat,
4-(N,N-Dimethylamino)-butyl-acrylat und -methacrylat,
3-(N,N-Dimethylamino)-propyl-acrylamid und -methacrylamid,
Triethanolamin-monoacrylat und -monomethacrylat,
2-(Dimethylaminoethyloxy)-ethyl-acrylat und -methacrylat,
2-Imidazolyl-ethyl-acrylat und -methacrylat,
2-Piperazinyl-ethyl-acrylat und -methacrylat,
2-Piperazinyl-ethyl-acrylamid und -methacrylamid,
N,N-Dimethylamino-neopentyl-acrylat und -methacrylat,
N,N-Dimethylamino-neopentyl-acrylamid und -methacrylamid,
(1,2,2,6,6-Pentamethyl-piperidyl-4)-acrylat und -methacrylat,
3-Morpholino-propyl-acrylamid und -methacrylamid, 2-Morpholino-ethyl-acrylat und -methacrylat,
2-(N,N-Dibutylamino)-ethyl-acrylat und -methacrylat,
4-Diethylamino-1-methyl-butyl-acrylamid und -methacrylamid,
Monomere mit quartären Ammoniumgruppen sind daraus durch Umsetzung mit Alkylierungsmitteln, wie Methylchlorid, Ethylbromid, u.ä. zugänglich.

Das Hüllmaterial kann das filmbildende Polymermaterial und das Polysaccharid im Gewichtsverhältnis von 2 : 1 bis 1 : 5, vorzugsweise 1 : 1 bis 1 : 4, enthalten. Darüberhinaus können übliche Zusätze, wie Füllstoffe, Farbstoffe, Pigmente, Weichmachungsmittel, Filmbildungshilfsmittel, Konservierungsmittel, Glanzmittel usw. darin enthalten sein.

Das Überzugsmittel enthält neben dem Polysaccharid und dem filmbildenden Polymeren eine flüssige Phase. Da Lösemittel, in denen sich die beiden erfindungswesentlichen Komponenten gemeinsam klar lösen, schon bei niedrigen Feststoffgehalten hohe Viskositäten ergeben und die Lösungen deshalb schwer zu verarbeiten sind, arbeitet man in der Praxis vorzugsweise mit Suspensionen oder Dispersionen der Komponenten in der flüssigen Phase, vorausgesetzt daß sie zu einem zusammenhängenden Film auftrocknen. Als flüssige Phase eignen sich z.B. niedere aliphatische Alkohole, wie Ethanol, Propanol, Butanol und besonders Isopropanol, sowie Ketone, wie Aceton, oder deren Gemische mit Wasser. In diesem Falle beträgt das bevorzugte Mischungsverhältnis von Alkohol oder Keton zu Wasser etwa 2 : 1 bis 1 : 2 Vol.-Teile.

Zweckmäßig stellt man das Überzugsmittel aus einer z.B. 20- bis 40-prozentigen wäßrigen Dispersion des filmbildenden Polymeren her, indem man sie mit dem gleichen Volumen des Alkohols verdünnt und eine Suspension des Polysaccharids in einem Wasser-Alkohol-Gemisch hinzufügt. Anschließend können gegebenenfalls weitere Bestandteile eingearbeitet werden. Wäßrige Dispersionen der filmbildenden Polymeren sind im Handel erhältlich oder können aus den pulverförmigen Polymeren durch Dispergieren in einer wäßrigen Phase erhalten werden; vgl. EP-B 181 515, EP 88 951.

Das flüssige Überzugsmittel hat vorzugsweise einen Gehalt an Feststoffen von 10 bis 30 Gew.-%, einschließlich aller Hilfsstoffe. Es kann nach gebräuchlichen galenischen Verfahren zum Pelletieren oder Dragieren verwendet oder auf Arzneiformen aufgebracht werden, z.B. durch Aufgießen oder Aufsprühen im Dragierkessel oder durch das Wirbelschichtverfahren. Die Viskosität läßt sich durch Verdünnen mit Lösungsmittel im Gemisch mit Wasser durch geeignete Wahl des Wasseranteils auf den erforderlichen Wert einstellen. Beim Verdunsten der flüssigen Phase erfolgt die Filmbildung.

Die orale Arzneiform kann jeden Wirkstoff enthalten, dessen Freisetzung im Colon aus pharmakologischen Gründen erwünscht ist. Dazu gehören z.B. 5-Aminosalicylsäure und Bisacodyl. Eine wichtige Klasse von Wirkstoffen, die erst im Colon freigesetzt werden dürfen, sind solche mit Peptid- oder Proteinstruktur. Sie würden in den oberen Darmabschnitten durch die körpereignen proteolytischen Enzyme abgebaut, bevor sie zur Wirkung kommen könnten. Im Colon ist der Gehalt an proteolytischen Enzymen so weit vermindert, daß eine ausreichende Einwirkungs- bzw. Resorptionszeit verbleibt. Ein Wirkstoff dieser Klasse ist z.B. Insulin.

Man kann Arzneiformen herstellen, indem man den Wirkstoff in der flüssigen Phase des Überzugsmittels löst und die Masse trocknet. In diesem Fall liegt der Wirkstoff in einer aus dem Hüllmaterial gebildeten Matrix in homogener Verteilung vor. Zur Herstellung von Matrixstrukturen können die Wirkstoffe gegebenenfalls zusammen mit weiteren Hilfsstoffen durch Befeuchten mit dem Überzugsmittel granuliert und anschließend verpreßt werden. Überwiegend geht man jedoch von Arzneiform-Kernen in Gestalt von Tabletten, Dragees, Sachets, Kapseln, Pellets, Streukügelchen, Granulaten, Kristallen oder Pulvern aus und überzieht diese, so daß der Wirkstoff und das Hüllmittel in getrennten Phasen vorliegen.

Gemäß einer bevorzugten Ausführungsform der Erfindung wird der Kern zunächst mit einer dünnen Isolierschicht aus einem magensaftresisten, im Darmsaft bei pH 5 bis 7 löslichen filmbildenden Polymermaterial überzogen, das so gewählt ist, daß es sich im Colon auch unter ungünstigen Bedingungen leicht löst. Geeignet sind Acrylpolymere mit einem verhältnismäßig hohen Anteil an Acryl- und/oder Methacrylsäure oder an Monomereinheiten mit quartären Ammoniumgruppen. Darüber wird die erfindungsgemäße Schicht des Hüllmaterials in einer Dicke von z.B. 10 bis 150 Mikrometer aufgetragen. Bei Tablettenkernen von 6 bis 10 mm Durchmesser entspricht dies in der Regel 1 bis 15 % des Feststoffgewichtes. Bei kleineren Kernen mit einer Größe von 0,1 bis 2 mm kann der Anteil des Hüllmaterials 5 bis 40 Gew.-% ausmachen. Die zeitliche Verzögerung der Wirkstofffreisetzung bis zur Einwirkung der glykosidischen Enzyme kann durch die Schichtdicke der voraussichtlichen Passagezeit durch den oberen Dünndarm bis zum Erreichen des Colons angepaßt werden. Eine Feinabstimmung der Schichtdicke durch Versuche in vivo ist empfehlenswert. Vorzugsweise wird diese Schicht noch mit einem magensaftresistenten Überzug in einer oder mehreren Schichten überzogen, der sich bereits beim Eintritt in den Dünndarm löst.

Größere Arzneiformen dieser Art werden als solche appliziert. Kleinere überzogene Teilchen können in Kapseln abgefüllt oder zu größeren Dosierungseinheiten verpreßt werden, wobei das Hüllmaterial gegebenenfalls eine zusammenhängende Matrix bildet, in der eine Vielzahl der ursprünglichen Kerne verteilt vorliegt. Gewünschtenfalls kann eine solche Einheit wiederum mit einem magensaftresistenten Überzug versehen werden.

Nach der Einnahme bleibt der magensaftresistente Überzug während des Durchgangs durch den Magen unverändert. Im oberen Dünndarm löst sich der magensaftresistente Überzug ab. Die nun freiliegende Schicht des erfindungsgemäßen Hüllmaterials beginnt zu quellen und kann dabei die 5- bis 10-fache Schichtdicke erreichen. Durch diese Quellung wird ein weitergehender Angriff auf die Isolierschicht oder - wenn keine Isolierschicht vorhanden ist - die vorzeitige Herauslösung des Wirkstoffes wenigstens 2 bis 4 Stunden lang verhindert. Durch Einarbeiten von sauren Puffersubstanzen, wie Citronensäure, Phosphorsäure, sauren Phosphaten, Alginsäure, Hyaluronsäure, Carboxymethylcellulose, Polyacrylsäure oder Methacrylsäure-Copolymeren, kann in der gequollenen Schicht über längere Zeit ein pH-Wert unter 5 aufrechterhalten werden, so daß die Verdauungssäfte des Darmes noch nicht wirksam werden. Auf diese Weise kann die Wirkungsdauer der erfindungsgemäßen Schicht weiter verlängert werden.

Die gequollene Schicht des Hüllmaterials wird erst im Colon bei pH-Werten von 6 bis 7,5 durch die von der Darmflora gebildteten glykosidischen Enzyme angegriffen, so daß sie mechanisch zerfällt und den Kern freigibt. Dieser kann nun ebenfalls schnell zerfallen und den Wirkstoff in kurzer Zeit freisetzen.

### BEISPIELE

### Beispiel 1

100 g Tabletten, die Methylenblau als Modellwirkstoff enthielten, wurden zunächst mit einem magensaftresistenten Überzug aus einem Methacrylsäure-Ethylacrylat-Copolymer 50 : 50 Gew.-% (Handelsprodukt EUDRAGIT^{(R)} L 30 D der Röhm Pharma GmbH) überzogen. Sie wurden dann in einem mit 30 UpM rotierenden Labor-Dragierkessel von 15 cm Durchmesser mit Warmluft auf 30°C vorgewärmt und dann mit 14 ml eines flüssigen Überzugsmittels gemäß der Erfindung in Portionen von je 0,2 bis 0,3 ml beschichtet. Gleichzeitig wurde mit Warmluft von 50°C getrocknet.

Das Überzugsmittel war zuvor auf folgende Weise bereitet worden: 2,22 g einer 30 %igen wäßrigen Dispersion eines redispergierten Copolymerisats aus Methylmethacrylat, Ethylacrylat und Trimethylammonio-ethyl-methacrylat-hydrochlorid, 60:30:10 Gew.-% (Handelsprodukt EUDRAGIT^{(R)} RL 30 D der Röhm Pharma GmbH) wurden mit 2 ml Isopropanol vermischt, wobei eine viskose Lösung entstand. Dazu wurde eine Aufschlämmung von 2,22 g Guar gum in 10 ml einer Mischung gleicher Volumenteile Isopropanol und Wasser gegeben und zu einer Suspension verrührt.

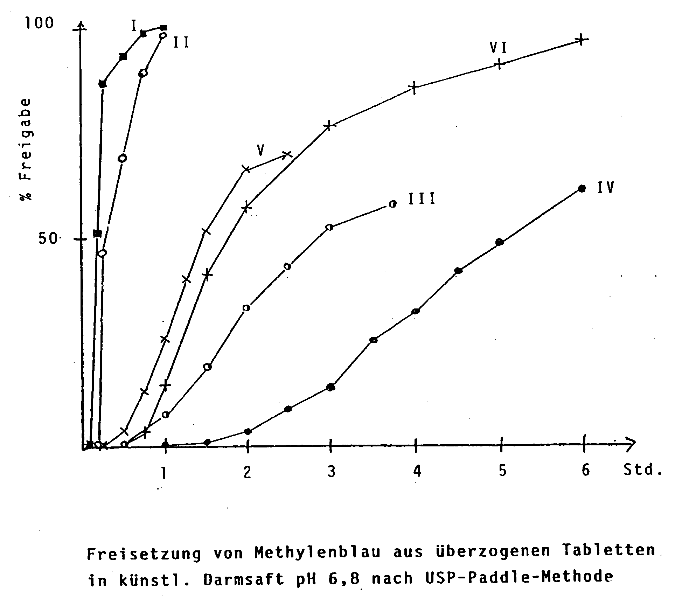

Nach Auftragen von einem Drittel, zwei Dritteln und der Gesamtmenge des Überzugsmittels wurden Proben der beschichteten Tabletten entnommen und über Nacht bei 40°C nachgetrocknet. Die Freisetzung des Wirkstoffes wurde in einem Paddle-Gerät nach USP bei 75 UpM und 37°C in jeweils 800 ml Flüssigkeit untersucht und colorimetrisch bestimmt. Die Ergebnisse sind in Figur 1 wiedergegeben.

Die Kurven I bis IV zeigen die verminderte Wirkstofffreisetzung in künstlichem Darmsatt von pH 6,8 in Abhängigkeit von der Dicke der erfindungsgemäßen Überzugsschicht. Die Zeit bis zur Freisetzung von 50 % des Wirkstoffes ist jeweils in Klammern angegeben.
- Kurve I:: ohne erfind.gem. Hüllmaterial (10 min)
- Kurve II:: 0,87 mg/qcm Hüllmaterial (15 min)
- Kurve III:: 1,70 mg/qcm Hüllmaterial (2 h, 40 min)
- Kurve IV:: 2,60 mg/qcm Hüllmaterial (5 h)
Die Kurven V und VI zeigen die Wirkstofffreisetzung in künstlichem Darmsatt beim gleichen pH-Wert und gleicher Temperatur, jedoch unter Zusatz von 100 mg eines Glykosidasen-Gemisches, enthaltend beta-Glucosidase, beta-Xylosidase und Galactomannanase.
- Kurve V:: 1,70 mg/qcm Hüllmaterial (1 h, 20 min)
- Kurve VI:: 2,60 mg/qcm Hüllmaterial (1 h, 40 min)
Aus den Kurven I bis IV geht die Freigabe-hemmende Wirkung des erfindungsgemäßen Überzugs in Abwesenheit von glykosidischen Enzymen und aus den Kurven V und VI die schnelle Freisetzung in Anwesenheit von glykosidischen Enzymen hervor.

### Beispiel 2

2,5 kg Tabletten von 7,0 mm Durchmesser und einem Einzelgewicht von 140 mg wurden in einem rotierenden Dragierkessel bei 20 U/min bewegt, auf 30°C erwärmt und durch Aufsprühen von 370 g EURDAGIT ® L 30 D (30-%ige wäßrige Dispersion eines Copolymerisates aus gleichen Teilen Ethylacrylat und Methacrylsäure) zusammen mit 27,0 g Talkum und 10,7 g Polywachs in 165 g Wasser magensaftresistent überzogen. Proben der Tabletten zeigten in künstlichem Magensaft nach 2 Stunden noch keinen Zerfall. Die Tabletten wurden nun im gleichen Dragierkessel bei 40 U/min mit der erfindungsgemäßen Schicht aus EUDRAGIT ® L 30 D und Guar Gum im Gewichtsverhältnis der Trokkensubstanzen von etwa 1 : 3 überzogen. Dazu wurden 300 g EUDRAGIT ® L 30 D in 300 g Isopropanol gelöst und mit einer Aufschlämmung von 250 g Guar Gum und 50 g Talkum in einer Mischung von je 800 g Isopropanol und Wasser vermischt und mit einer Sprühpistole innerhalb von 155 min kontinuierlich auf die Tabletten aufgesprüht und gleichzeitig bei einer Kerntemperatur von 30°C mit Warmluft getrocknet. Die 2 Stunden bei 40°C im Umlufttrockenschrank nachgetrockneten Tabletten zeigen im USP-Paddle-Gerät in künstlichem Darmsaft pH 6,8 eine verzögerte Wirkstoffabgabe. Nach 60 min sind die Tabletten aufgequollen, aber erst 19 % des Wirkstoffes freigesetzt. Bei Zugabe von 100 mg einer Glactomannanase zum künstlichen Darmsaft wurden schon nach 45 min über 80 % des Wirkstoffes freigesetzt.

### Beispiel 3

Der Versuch nach Beispiel 2 wurde so modifizizert, daß die Tabletten zunächst nicht magensaftresistent, sondern nur mit einer inneren Isolierschicht aus 93 g EUDRAGIT ® L 30 D (30 g TS) zusammen mit 10 g Talkum und 2,8 g Triethylcitrat als Weichmacher überzogen wurden. Diese Tabletten zerfallen in Magensaft oder Wasser innerhalb von 30 min. Anschließend wurde eine Zwischenschicht aus 280 g EUDRAGIT ® L 30 D, 232 g Guar Gum, 47 g Talkum in Isopropanol/Wasser in gleicher Weise aufgebracht. Darüber wurde noch eine äußere, magensaftresistente Schicht aus 480 g EUDRAGIT ® L 30 D, 54 g Talkum und 14,4 g Triethylcitrat aufgebracht. Diese Tabletten zerfallen im USP-Paddle-Gerät in künstlichem Magensafft innerhalb von 2 Stunden nicht und setzen nur 1,7 % des Wirkstoffes frei, sind also magensaftresistent. In künstlichem Darmsaft pH 6,8 ist die Wirkstoffabgabe zunächst verzögert (13,9 % innerhalb von 45 min) mit Zusatz von Galactomannanase jedoch sehr schnell (nach 30 min über 50 %).

### Beispiel 4

Beispiel 1 wurde mit 2,5 kg Tabletten in einem Dragierkessel von 35 cm Durchmesser unter sonst gleichen Arbeitsbedingungen wiederholt. Die quellbare Zwischenschicht wurde jedoch aus 290 g EUDRAGIT ® RL 30 D (30-%ige wäßrige Dispersion eines Copolymerisates aus 30 Teilen Ethylacrylat, 60 Teilen Methylmethacrylat und 10 Teilen Trimethylammonioethyl-methacrylat-chlorid), 320 g Guar Gum, 70 g Talkum, 780 g Isopropanol und 780 g Wasser formuliert. Das Gewichtsverhältnis der Trockensubstanz aus EUDRAGIT ® RL 30 D zu Guar Gum betrug hier etwa 1 : 4. In künstlichem Darmsaft pH 6,8 werden aus diesen Tabletten innerhalb 3,5 Stunden weniger als 1 % und in 4 Stunden nur 7 % Wirkstoff freigesetzt. Dagegen gehen bei Zusatz von 100 mg Galactomannanase pro 900 ml des gleichen künstlichen Darmsaftes nach 3 Stunden schon 33 % des Wirkstoffes in Lösung.

### Beispiel 5

2,5 kg Tabletten mit 7,0 mm Durchmesser und 140 mg Einzelgewicht wurden ohne Vorisolierung direkt mit einer Mischung aus 140 g EUDRAGIT ® S 100 (Copolymerisat aus 70 Gewichtsteilen Methylmethacrylat und 30 Gewichtsteilen Methacrylsäure), 346 g Guar Gum, 74 g Talkum, 1 786 g Isopropanol und 1 312 g Wasser als Lösungs- und Dispergiermittel überzogen. Dazu wurden die Tabletten in einem rotierenden Dragierkessel von 35 cm Durchmesser mit 30 - 40 U/min bewegt und ständig Warmluft von 50°C eingeblasen, um die Temperatur der Kerne bei 30°C zu halten. Zuletzt wurden die Kerne bei 40°C 2 Stunden im Umlufttrockenschrank getrocknet. Im USP-Paddle-Gerät mit 900 ml künstlichem Darmsaft pH 6,8 wird nach 30 min weniger als 2 % Wirkstoff freigesetzt, während bei Zugabe von 100 mg Glactomannanase nach 30 min 55 % und nach 60 min 90 % gelöst sind,

### Beispiel 6

2 kg Prednisolon-Pellets mit einem Wirkstoffgehalt von 5 % und 0,8 - 1,2 mm Partikeldurchmesser wurden in einem Wirbelschichtgerät (Glatt GPCG1) zunächst mit 330 g EUDRAGIT ® L 30 D (30 %-ige wäßrige Dispersion eines Copolymerisates aus gleichen Teilen Ethylacrylat und Methacrylsäure) unter Zusatz von 10 g Triethylcitrat als Weichmacher durch Aufsprühen in einem Warmluftstrom von 30 - 40°C vorisoliert. Dann wurden 300 g Guar Gum und 60 g Talkum in einem Gemisch aus je 1 kg Isopropanol und 1 kg Wasser suspendiert und mit einer Lösung von 330 g EUDRAGIT ® RL 30 D (30 %-ige wäßrige Dispersion eines Copolymerisates aus 30 Teilen Ethylacrylat, 60 Teilen Methylmethacrylat und 10 Teilen Trimethylammonioethyl methacrylat-chlorid) in 330 g Isopropanol vermischt. Die Mischung wurde mit einem Ultra-Turrax homogenisiert und bei 35 - 45°C Zulufttemperatur auf die im gleichen Wirbelschichtgerät befindlichen, vorisolierten Kerne innerhalb von 125 min aufgesprüht. Die überzogenen Pellets wurden über Nacht bei 40°C auf Horden nachgetrocknet.

### Beispiel 7

2,5 kg Theophyllin-Pulver einer Korngröße von 0,05 - 0,07 mm wurde in einer Rührschüssel (Stephan-Mixer) mit 200 - 500 U/min bewegt. 270 g Guar Gum wurden in einem Gemisch von 270 g Isopropanol und 270 g Wasser suspendiert und mit einer Lösung von 300 g EUDRAGIT ® RS 30 D (30 %-ige wäßrige Dispersion eines Copolymerisates aus 30 Teilen Ethylacrylat, 65 Teilen Methylmethacrylat und 5 Teilen Trimethylammonioethyl methacrylat-chlorid) in 300 g Isopropanol vermischt. Diese Mischung wurde in Portionen zu je 100 - 200 ml in das Theophyllin-Pulver unter Rühren eingetragen und die feuchte Masse durch ein Sieb mit 1,0 mm Maschenweite passiert. Das Granulat wurde 24 Stunden bei 40°C getrocknet und unter Zusatz von 20 % Cellulose-Pulver (Avicel PH 102) und 0,5 % Magnesiumstearat zu Tabletten verpreßt.

### Beispiel 8

Beispiel 7 wurde in der Weise modifiziert, daß 270 g Guar Gum trocken in 2,5 kg Theophyllinpulver eingemischt und dann allein mit der Lösung von EUDRAGIT ® RL 30 D in 300 g Isopropanol befeuchtet und getrocknet wurde. Dieses zunächst noch sehr feine Granulat wurde nun nochmals mit einer Mischung von 350 g Isopropanol und 350 g Wasser befeuchtet, durch ein 1 mm-Sieb gedrückt und wieder wie in Beispiel 7 getrocknet.

## Patentansprüche (Patentansprüche für folgende(n) Vertragsstaat(en): AT, BE, CH, LI, DE, DK, FR, GB, IT, NL, SE)

1. Zur Wirkstofffreigabe im Colon bestimmte orale Arzneiform, enthaltend Wenigstens einen Wirkstoff und wenigstens ein, den Wirkstoff umschließendes Hüllmaterial, das ein im Colon abbaubares Polysaccharid, das wesentliche Anteile an Galactose- und Mannose-Einheiten aufweist, enthält, ausgenommen Mikrokapseln, die in dem Hüllmaterial eine ölige Flüssigkeit eingeschlossen enthalten,
dadurch gekennzeichnet,
daß das Hüllmaterial in Mischung mit dem Polysaccharid ein filmbildendes Polymermaterial enthält.

2. Orale Arzneiform nach Anspruch 1, dadurch gekennzeichnet, daß das Polysaccharid Johannisbrotkernmehl oder Guar Gum ist.

3. Orale Arzneiform nach Anspruch 1, dadurch gekennzeichnet, daß das filmbildende Polymermaterial hydrophile Gruppen enthält.

4. Orale Arzneiform nach einem der Ansprüche 1 bis 3, dadurch gekennzeichnet, daß das filmbildende Polymermaterial ein Acrylpolymer ist.

5. Orale Arzneiform nach Anspruch 4, dadurch gekennzeichnet, daß das Acrylpolymer zu 30 bis 100 Gew.-% aus Monomereinheiten von niederen Alkylestern der Acryl- und/oder Methacrylsäure und gegebenenfalls bis zu 70 Gew.-% aus funktionalen Monomereinheiten von äthylenisch ungesättigten, radikalisch polymerisierbaren Monomeren mit einer Carboxyl-, Carboxylat-, tertiären Amino-, quartären Ammonium- oder Hydroxylgruppe aufgebaut ist.

6. Orale Arzneiform nach Anspruch 5, dadurch gekennzeichnet, daß sich die funktionalen Monomereinheiten Von Acryl- und/oder Methacrylsäure oder Derivaten davon ableiten.

7. Orale Arzneiform nach einem oder mehreren der Ansprüche 1 bis 6, dadurch gekennzeichnet, daß das Hüllmaterial das filmbildende Poly-mermaterial und das Polysaccharid im Gewichtsverhältnis von 2 : 1 bis 1 : 5 enthält.

8. Orale Arzneiform nach Anspruch 7, dadurch gekennzeichnet, daß das Hüllmaterial das filmbildende Polymermaterial und das Polysaccharid im Gewichtsverhältnis von 1 : 1 bis 1 : 4 enthält.

9. Orale Arzneiform nach einem oder mehreren der Ansprüche 1 bis 8, dadurch gekennzeichnet, daß das Hüllmaterial einen den Wirkstoff enthaltenden Kern in Form eines Überzugs umschließt.

10. Orale Arzneiform nach Anspruch 7, dadurch gekennzeichnet, daß der Kern unter dem Hüllmaterial eine Isolierschicht aus einem darmsaftlöslichen Überzugsmaterial enthält.

11. Orale Arzneiform nach einem oder mehreren der Ansprüche 1 bis 8, dadurch gekennzeichnet, daß das Hüllmaterial eine Matrix bildet, in der der Wirkstoff homogen oder in Form von Kristallen oder eines Granulats verteilt ist.

12. Orale Arzneiform nach einem oder mehreren der Ansprüche 1 bis 11, dadurch gekennzeichnet, daß sie über dem Hüllmaterial einen Überzug aus einem magensaftresistenten, darmsaftlöslichen Überzugsmaterial enthält.

13. Orale Arzneiform nach einem oder mehreren der Ansprüche 1 bis 12 in Gestalt von Tabletten, Dragees, Sachets, Kapseln, Pellets, Streukügelchen oder Granulaten.

14. Überzugsmittel für Arzneimittel, enthaltend eine flüssige Phase, ein im Colon abbaubares Polysaccharid, das wesentliche Anteile an Galactose- und Mannose-Einheiten aufweist, und ein filmbildendes Polymermaterial, das in Form von Latexteilchen in der flüssigen Phase dispergiert ist.

15. Überzugsmittel nach Anspruch 14, dadurch gekennzeichnet, daß es eine flüssige Phase aus einem wassermischbaren niederen aliphatischen Alkohol oder Keton und gegebenenfalls Wasser enthält.

## Patentansprüche (Patentansprüche für folgende(n) Vertragsstaat(en): ES)

1. Verfahren zur Herstellung einer zur Wirkstofffreigabe im Colon bestimmten oralen Arzneiform, enthaltend wenigstens einen Wirkstoff und wenigstens ein, den Wirkstoff umschließendes Hüllmaterial, das ein im Colon abbaubares Polysaccharid, das wesentliche Anteile an Galactose- und Mannose-Einheiten aufweist, enthält, ausgenommen Mikrokapseln, die in dem Hüllmaterial eine ölige Flüssigkeit eingeschlossen enthalten,
dadurch gekennzeichnet,
daß als Hüllmaterial in Mischung mit dem Polysaccharid ein filmbildendes Polymermaterial eingesetzt wird.

2. Verfahren nach Anspruch 1, dadurch gekennzeichnet, daß das Polysaccharid Johannisbrotkernmehl oder Guar Gum ist.

3. Verfahren nach Anspruch 1, dadurch gekennzeichnet, daß ein filmbildendes Polymermaterial eingesetzt wird, das hydrophile Gruppen enthält.

4. Verfahren nach einem der Ansprüche 1 bis 3, dadurch gekennzeichnet, daß das filmbildende Polymermaterial ein Acrylpolymer ist.

5. Verfahren nach Anspruch 4, dadurch gekennzeichnet, daß das Acrylpolymer zu 30 bis 100 Gew.-% aus Monomereinheiten von niederen Alkylestern der Acryl- und/oder Methacrylsäure und gegebenenfalls bis zu 70 Gew.-% aus funktionalen Monomereinheiten von äthylenisch ungesättigten, radikalisch polymerisierbaren Monomeren mit einer Carboxyl-, Carboxylat-, tertiären Amino-, quartären Ammonium- oder Hydroxylgruppe aufgebaut ist.

6. Verfahren nach Anspruch 5, dadurch gekennzeichnet, daß sich die funktionalen Monomereinheiten von Acryl- und/oder Methacrylsäure oder Derivaten davon ableiten.

7. Verfahren nach einem oder mehreren der Ansprüche 1 bis 6, dadurch gekennzeichnet, daß das Hüllmaterial das filmbildende Polymermaterial und das Polysaccharid im Gewichtsverhältnis von 2 : 1 bis 1 : 5 enthält.

8. Verfahren nach Anspruch 7, dadurch gekennzeichnet, daß das Hüllmaterial das filmbildende Polymermaterial und das Polysaccharid im Gewichtsverhältnis von 1 : 1 bis 1 : 4 enthält.

9. Verfahren nach einem oder mehreren der Ansprüche 1 bis 8, dadurch gekennzeichnet, daß auf einem den Wirkstoff enthaltenden Kern ein Überzugs des Hüllmaterials erzeugt wird.

10. Verfahren nach Anspruch 7, dadurch gekennzeichnet, daß der Kern vor der Beschichtung mit dem Hüllmaterial mit einer Isolierschicht aus einem darmsaftlöslichen Überzugsmaterial überzogen wird.

11. Verfahren nach einem oder mehreren der Ansprüche 1 bis 8, dadurch gekennzeichnet, daß aus dem Hüllmaterial eine Matrix gebildet wird, in der der Wirkstoff homogen oder in Form von Kristallen oder eines Granulats verteilt ist.

12. Verfahren nach einem oder mehreren der Ansprüche 1 bis 11, dadurch gekennzeichnet, daß über dem Hüllmaterial ein Überzug aus einem magensaftresistenten, darmsaftlöslichen Überzugsmaterial erzeugt wird.

13. Verfahren nach einem oder mehreren der Ansprüche 1 bis 12, dadurch gekennzeichnet, daß orale Arzneiformen in Gestalt von Tabletten, Dragees, Sachets, Kapseln, Pellets, Streukügelchen oder Granulaten erzeugt werden.

14. Verfahren nach einem oder mehreren der Ansprüche 1 bis 13, dadurch gekennzeichnet, daß ein Überzugsmittel für Arzneimittel, enthaltend eine flüssige Phase, ein im Colon abbaubares Polysaccharid, das wesentliche Anteile an Galactose- und Mannose-Einheiten aufweist, enthält und ein filmbildendes Polymermaterial, das in Form von Latexteilchen in der flüssigen Phase dispergiert ist, eingesetzt wird.

15. Verfahren nach Anspruch 14, dadurch gekennzeichnet, daß das Überzugsmittel eine flüssige Phase aus einem wassermischbaren niederen aliphatischen Alkohol oder Keton und gegebenenfalls Wasser enthält.

## Claims (Claims for the following Contracting State(s): AT, BE, CH, LI, DE, DK, FR, GB, IT, NL, SE)

1. An oral pharmaceutical composition intended to release an active substance in the colon, containing at least an active substance and at least a covering material which encloses the active substance and which contains a polysaccharide degradable in the colon, substantially containing proportions of galactose and mannose units, with the exception of micro capsules which contain an oily fluid enclosed in the covering material, characterised in that the covering material contains a film-forming polymer material mixed with the polysaccharide.

2. An oral pharmaceutical composition according to claim 1, characterised in that the polysaccharide is powdered carob bean or guar gum.

3. An oral pharmaceutical composition according to claim 1, characterised in that the film-forming polymer material contains hydrophilic groups.

4. An oral pharmaceutical composition according to claims 1 to 3, characterised in that the film-forming polymer material is an acrylic polymer.

5. An oral pharmaceutical composition according to claim 4, characterised in that the acrylic polymer comprises 30 to 100 wt.% of monomer units of lower alkyl esters of acrylic and/or methacrylic acid and, optionally, up to 70 wt.% of functionalised monomer units of ethylenically unsaturated, radically polymerisable monomers with a carboxyl, carboxylate, tertiary amino, quarternary ammonium or hydroxyl group functionality.

6. An oral pharmaceutical composition according to claim 5, characterised in that the functionalised monomer units are derived from acrylic and/or methacrylic acid or derivatives thereof.

7. An oral pharmaceutical composition according to one or more of claims 1 to 6, characterised in that the covering material contains the film-forming polymer material and the polysaccharide in a weight ratio of 2 : 1 to 1 : 5.

8. An oral pharmaceutical composition according to claim 7, characterised in that the covering material contains the film-forming polymer material and the polysaccharide in a weight ratio of 1 : 1 to 1 : 4.

9. An oral pharmaceutical composition according to one or more of claims 1 to 8, characterised in that the covering material, in the form of a cover, encloses the core containing the active substance.

10. An oral pharmaceutical composition according to claim 7, characterised in that the core contains an insulating layer, consisting of a coating material which dissolves in the intestinal juice, under the covering material.

11. An oral pharmaceutical composition according to one or more of claims 1 to 8, characterised in that the covering material forms a matrix in which the active substance is distributed either homogenously or in the form of crystals or a granulate.

12. An oral pharmaceutical composition according to one or more of claims 1 to 11, characterised in that it contains a coating on top of the covering material, comprising a coating material resistant to gastric juice and soluble in intestinal juice.

13. An oral pharmaceutical composition according to one or more of claims 1 to 12, in the form of plain and coated tablets, sachets, capsules, pellets, dispersible pellets or granulates.

14. A coating material for pharmaceutical compositions containing a liquid phase, a polysaccharide degradable in the colon which substantially contains proportions of galactose and mannose units, and a film-forming polymer material which is dispersed in the liquid phase in the form of latex particles.

15. A coating material according to claim 14, characterised in that it contains a liquid phase comprising a water-miscible lower aliphatic alcohol or ketone and, optionally, water.

## Claims (Claims for the following Contracting State(s): ES)

1. A process for the preparation of oral pharmaceutical composition intended to release an active substance in the colon, said composition containing at least an active substance and at least a covering material which encloses the active substance and which contains a polysaccharide degradable in the colon, substantially containing proportions of galactose and mannose units, with the exception of micro capsules which contain an oily fluid enclosed in the covering material, characterised in that a film-forming polymer material mixed with the polysaccharide is used to form the covering material.

2. A process according to claim 1, characterised in that the polysaccharide is powdered carob bean or guar gum.

3. A process according to claim 1, characterised in that the film-forming polymer material used contains hydrophilic groups.

4. A process according to claim 1 or 3, characterised in that the film-forming polymer material is an acrylic polymer.

5. A process according to claim 4, characterised in that the acrylic polymer is synthesised, in an amount of from 30 to 100 wt.%, from monomer units of lower alkyl esters of acrylic and/or methacrylic acid and, optionally, in an amount of up to 70 wt.%, from functionalised monomer units of ethylenically unsaturated, radically polymerisable monomers with a carboxyl, carboxylate, tertiary amino, quarternary ammonium or hydroxyl group functionally.

6. A process according to claim 5, characterised in that the functionalized monomer units are derived from acrylic and/or methacrylic acid or derivatives thereof.

7. A process according to one or more of claims 1 to 6, characterised in that the covering material contains the film-forming polymer material and the polysaccharide in a weight ratio of from 2 : 1 to 1 : 5.

8. A process according to claim 7, characterised in that the covering material contains the film-forming polymer material and the polysaccharide in a weight ratio of from 1 : 1 to 1 : 4.

9. A process according to one or more of claims 1 to 8, characterized in that the covering material is produced, in the form of a cover enclosing the core containing the active substance.

10. A process according to claim 7, characterized in that before the core is coated with the covering material, an insulating layer which dissolves in the intestinal juice, is applied to the core.

11. A process according to one or more of claims 1 to 8, characterized in that the covering material used forms a matrix in which the active substance is distributed either homogenously or in the form of crystals or a granule.

12. A process according to one or more of claims 1 to 11, characterised in that a coating material resistant to gastric juice and soluble in intestinal juice is used on top of the covering material.

13. A process according to one or more of claims 1 to 12, characterised in that the oral pharmaceutical composition is produced in the form of plain and coated tablets, sachets, capsules, pellets, dispersible pellets or granules.

14. A process according to one or more of claims 1 to 13, characterised in that a coating material for pharmaceutical compositions containing a liquid phase, a polysaccharide degradable in the colon and substantially containing proportions of galactose and mannose units, and a film-forming polymer material which is dispersed in the liquid phase in the form of latex particles is used.

15. A process according to claim 14, characterized in that the coating material used contains a liquid phase consisting of a water-miscible lower aliphatic alcohol or ketone and, optionally, water.

## Revendications (Revendications pour l'(les) Etat(s) contractant(s) suivant(s): AT, BE, CH, LI, DE, DK, FR, GB, IT, NL, SE)

1. Forme médicamenteuse orale destinée à libérer la substance active dans le côlon, contenant au moins une substance active et au moins une matière d'enrobage qui entoure la substance active et qui contient un polysaccharide dégradable dans le côlon comportant des proportions importantes de motifs galactose et mannose, à l'exclusion de microcapsules qui contiennent un liquide huileux inclus dans la matière d'enrobage, caractérisée en ce que la matière d'enrobage contient, en mélange avec le polysaccharide, une matière polymère filmogène.

2. Forme médicamenteuse orale selon la revendication 1, caractérisée en ce que le polysaccharide est de la farine de caroube ou de la gomme de guar.

3. Forme médicamenteuse orale selon la revendication 1, caractérisée en ce que la matière polymère filmogène contient des groupements hydrophiles.

4. Forme médicamenteuse orale selon l'une quelconque des revendications 1 à 3, caractérisée en ce que la matière polymère filmogène est un polymère acrylique.

5. Forme médicamenteuse orale selon la revendication 4, caractérisée en ce que le polymère acrylique est composé, pour 30 à 100% en poids, de motifs monomères d'esters alkyliques inférieurs de l'acide acrylique et/'ou méthacrylique et éventuellement, dans une proportion pouvant aller jusqu'à 70% en poids, de motifs monomères fonctionnels de monomères à insaturation éthylénique, susceptibles de polymérisation radicalaire, renfermant un groupement carboxyle, carboxylate, amino tertiaire, ammonium quaternaire ou hydroxy.

6. Forme médicamenteuse orale selon la revendication 5, caractérisée en ce que les motifs monomères fonctionnels dérivent d'acide acrylique et/ou méthacrylique ou de dérivés de ceux-ci.

7. Forme médicamenteuse orale selon l'une quelconque des revendications 1 à 6, caractérisée en ce que la matière d'enrobage contient la matière polymère filmogène et le polysaccharide dans un rapport en poids de 2 : 1 à 1 : 5.

8. Forme médicamenteuse orale selon la revendication 7, caractérisée en ce que la matière d'enrobage contient la matière polymère filmogène et le polysaccharide dans un rapport en poids de 1 : 1 à 1 : 4.

9. Forme médicamenteuse orale selon l'une quelconque des revendications 1 à 8, caractérisée en ce que la matière d'enrobage engaine sous forme d'un revêtement un noyau qui contient la substance active.

10. Forme médicamenteuse orale selon la revendication 7, caractérisée en ce que le noyau contient, au-dessous de la matière d'enrobage, une couche isolante en une matière de revêtement soluble dans le suc intestinal.

11. Forme médicamenteuse orale selon l'une quelconque des revendications 1 à 8, caractérisée en ce que la matière d'enrobage forme une matrice dans laquelle la substance active est distribuée de façon homogène ou sous forme de cristaux ou d'un granulé.

12. Forme médicamenteuse orale selon l'une quelconque des revendications 1 à 11, caractérisée en ce qu'elle contient, au-dessus de la matière d'enrobage, un enduit en une matière de revêtement résistante au suc gastrique et soluble dans le suc intestinal.

13. Forme médicamenteuse orale selon l'une quelconque des revendications 1 à 12, sous forme de comprimés, de dragées, de capsules, de pilules, de perles ou de granulés.

14. Produit de revêtement pour médicaments, contenant une phase liquide, un polysaccharide dégradable dans le côlon comportant des proportions importantes de motifs galactose et mannose, et une matière polymère filmogène qui est dispersée dans la phase liquide sous forme de particules de latex.

15. Produit de revêtement selon la revendication 14, caractérisé en ce qu'il contient une phase liquide composée d'un alcool ou d'une cétone aliphatique inférieur miscible à l'eau et éventuellement d'eau.

## Revendications (Revendications pour l'(les) Etat(s) contractant(s) suivant(s): ES)

1. Procédé de préparation d'une forme médicamenteuse orale destinée à libérer la substance active dans le côlon, contenant au moins une substance active et au moins une matière d'enrobage qui entoure la substance active et qui contient un polysaccharide dégradable dans le côlon comportant des proportions importantes de motifs galactose et mannose, à l'exclusion de microcapsules qui contiennent un liquide huileux inclus dans la matière d'enrobage, caractérisé en ce que la matière d'enrobage contient, en mélange avec le polysaccharide, une matière polymère filmogène.

2. Procédé selon la revendication 1, caractérisé en ce que le polysaccharide est de la farine de caroube ou de la gomme de guar.

3. Procédé selon la revendication 1, caractérisé en ce que la matière polymère filmogène contient des groupements hydrophiles.

4. Procédé selon l'une quelconque des revendications 1 à 3, caractérisé en ce que la matière polymère filmogène est un polymère acrylique.

5. Procédé selon la revendication 4, caractérisé en ce que le polymère acrylique est composé, pour 30 à 100% en poids, de motifs monomères d'esters alkyliques inférieurs de l'acide acrylique et/ou méthacrylique et éventuellement, dans une proportion pouvant aller jusqu'à 70% en poids, de motifs monomères fonctionnels de monomères à insaturation éthylénique, susceptibles de polymérisation radicalaire, renfermant un groupement carboxyle, carboxylate, amino tertiaire, ammonium quaternaire ou hydroxy.

6. Procédé selon la revendication 5, caractérisé en ce que les motifs monomères fonctionnels dérivent d'acide acrylique et/ou méthacrylique ou de dérivés de ceux-ci.

7. Procédé selon l'une quelconque des revendications 1 à 6, caractérisé en ce que la matière d'enrobage contient la matière polymère filmogène et le polysaccharide dans un rapport en poids de 2 : 1 à 1 : 5.

8. Procédé selon la revendication 7, caractérisé en ce que la matière d'enrobage contient la matière polymère filmogène et le polysaccharide dans un rapport en poids de 1 : 1 à 1 : 4.

9. Procédé selon l'une quelconque des revendications 1 à 8, caractérisé en ce que la matière d'enrobage engaine sous forme d'un revêtement un noyau qui contient la substance active.

10. Procédé selon la revendication 7, caractérisé en ce que le noyau contient, au-dessous de la matière d'enrobage, une couche isolante en une matière de revêtement soluble dans le suc intestinal.

11. Procédé selon l'une quelconque des revendications 1 à 8, caractérisé en ce que la matière d'enrobage forme une matrice dans laquelle la substance active est distribuée de façon homogène ou sous forme de cristaux ou d'un granulé.

12. Procédé selon l'une quelconque des revendications 1 à 11, caractérisé en ce qu'on dispose, au-dessus de la matière d'enrobage, un enduit en une matière de revêtement résistante au suc gastrique et soluble dans le suc intestinal.

13. Procédé selon l'une quelconque des revendications 1 à 12, caractérisé en ce que la forme médicamenteuse orale est sous forme de comprimés, de dragées, de capsules, de pilules, de perles ou de granulés.

14. Procédé selon l'une quelconque des revendications 1 à 13, caractérisé en ce que l'on utilise un produit de revêtement pour médicaments, contenant une phase liquide, un polysaccharide dégradable dans le côlon comportant des proportions importantes de motifs galactose et mannose, et une matière polymère filmogène qui est dispersée dans la phase liquide sous forme de particules de latex.

15. Procédé selon la revendication 14, caractérisé en ce que le produit de revêtement contient une phase liquide composée d'un alcool ou d'une cétone aliphatique inférieur miscible à l'eau et éventuellement d'eau.
